# EUROPEAN PATENT SPECIFICATION

(11) **EP 3 548 894 B1**
(45) Date of publication and mention of the grant of the patent: **20.10.2021**
(21) Application number: 17811528.3
(22) Date of filing: 01.12.2017
(51) Int. Cl.: G01N 33/574

(54) **METHODS AND COMPOSITIONS FOR DIAGNOSING RENAL CELL CARCINOMA**
VERFAHREN UND ZUSAMMENSETZUNGEN ZUR DIAGNOSE EINES NIERENZELLKARZINOMS
PROCÉDÉS ET COMPOSITIONS PERMETTANT DE DIAGNOSTIQUER LE CARCINOME CELLULAIRE RÉNAL

(30) Priority: 02.12.2016 EP 16306611
(43) Date of publication of application: 09.10.2019
(73) Proprietor: INSERM (Institut National de la Santé et de la Recherche Médicale), 75013 Paris (FR); Université Paris-Saclay, 91190 Gif-sur-Yvette (FR)
(72) Inventor: EID, Pierre, 94807 Villejuif (FR); AZZI-HATEM, Sandy, 94807 Villejuif (FR)
(74) Representative: Inserm Transfert
(86) International application number: PCT/EP2017/081255
(87) International publication number: WO 2018/100190

(56) References cited:
- WO-A1-2013/052631
- US-A1- 2010 104 570
- US-A1- 2013 225 656
- PAL SUMANTA K ET AL: "Differentiating mTOR inhibitors in renal cell carcinoma", CANCER TREATMENT REVIEWS, vol. 39, no. 7, November 2013 (2013-11), pages 709-719, XP028691023, ISSN: 0305-7372, DOI: 10.1016/J.CTRV.2012.12.015
- A. L. PASTORE ET AL: "Serum and Urine Biomarkers for Human Renal Cell Carcinoma", DISEASE MARKERS., vol. 2015, 1 April 2015 (2015-04-01), pages 1-9, XP055357489, GB ISSN: 0278-0240, DOI: 10.1155/2015/251403

## Description

### FIELD OF THE INVENTION:

The invention relates to the field of nephrology. More particularly, the invention relates to a biomarker of renal cell carcinoma.

### BACKGROUND OF THE INVENTION:

Renal cell carcinoma (RCC) is the most common type of kidney cancer and the deadliest of genitourinary tumours in the Western population (Cairns P, 2010). Many risk factors have been identified and include gender (higher risk in males), age (more frequent in elderly), genetic mutations and life behavior (smoking, overweight, etc.) (Rodrigues D, 2016). Renal cell cancer starts in the lining of the tubules and counts different histologic subtypes amongst which the clear cell renal carcinoma (ccRCC, most frequent type with almost 80% of cases), papillary renal cell cancer, chromophobe renal cell cancer and other less common types (Moch H, 2016). Most people with RCC show symptoms such as hematuria, pain in the flank, a mass in the abdomen together with an important weight loss and fever. However, most of these symptoms are not exclusive to RCC and do not occur until advanced stages of the disease. Indeed, RCC develops asymptomatically for a long period of time and is often diagnosed incidentally. Therefore, most of the patients are taken in charge very late with the disease being already well spread, and very often at metastatic stage, with poor prognosis (Lam JS, 2005).

Renal cell carcinoma is very resistant to conventional radiation and chemotherapy and poorly sensitive to immunotherapy. Highly vascularized due to a loss of function of the von Hippel-Lindau gene (VHL) (Ohh et al. 2003), RCC respond well to anti-angiogenic molecules such as VEGF, protein tyrosine kinases and mTOR inhibitors. While these molecules have enabled great progress in terms of survival and quality of life, they show important side effects (i.e. blood pressure and digestive toxicity). Furthermore, after a first line of treatment, patients acquire resistance to these drugs and no significant increase in remission's rate is showed (Hamnvik et al 2015, Escudier et al 2012). For these reasons, it is unlikely that existing therapeutics will ever cure most patients with renal cancer, and partial or total nephrectomy remains the mainstay of treatment. However, to be efficiently operable, RCC must be confined to the kidney or sparsely extended to surrounding tissues. This implies early detection of the disease. Currently, diagnosis is restricted to imaging and biopsies techniques. Indeed there is no single biomarker for RCC. If numerous studies have reported many candidates (CA-IX, TRAF-1, SAA, KIM-1, AQP_1 and PLIN2) (Rajandram 2014, Fischer K 2012, Morrissey JJ 2011, Morrissey JJ 2014, Morrissey JJ 2015), these proteins remain detectable in advanced stage of RCC, and the quest for early stage biomarkers remains an open conundrum. Thus, development of new early detection approaches is at the utmost importance, as it will allow an improvement of patient care and a better overall survival rate. On another level, RCC comprises a subpopulation of primitive cells (Bussolati et al. 2008), the so-called cancer stem-like cells (CSC) that constitute a reservoir of self-sustaining cells with the exclusive ability to initiate, maintain and renew the tumour (Singh et al., 2003). Whether these CSC arise from normal stem cells or more differentiated ones is not known. However, regardless of tumour ontogeny, they might participate to tumour resistance to radiotherapies, recurrence and aggressiveness (Bao et al., 2006). Pastore et al.; "Serum and Urine Biomarkers for Human Renal Cell Carcinoma",DISEASE MARKERS., vol. 2015, 1 April 2015 (2015-04-01), reviews biomarkers for renal cell carcinoma in serum and urine.

### SUMMARY OF THE INVENTION:

The present invention relates to a method for diagnosing renal cell carcinoma (RCC) in a subject comprising the steps of: i) measuring the expression level of soluble SEMA7A in a fluid sample; ii) comparing the expression measured at step i) with its predetermined reference value, and iii) concluding that the subject suffers from RCC when the expression level of SEMA7A is higher than its predetermined reference value or concluding that the subject does not suffer from RCC when the expression level of SEMA7A is lower than its predetermined reference value. In particular, the present invention is defined by the claims.

### DETAILED DESCRIPTION OF THE INVENTION:

Inventors have performed extensive mass spectrometry analysis of two renal cancer cells secretome in order to identify potential circulating biomarkers. Semaphorin-7A (SEMA7A) was found in both secretome and its secretion confirmed by ELISA assay in renal cancer stem (CSC) and non-stem cells (ACHN). They further assessed SEMA7A detection in plasma of patients with RCC. They found that SEMA7A was detected in all patients despite the disease ontogeny. Moreover, SEMA7A levels were significantly reduced in patients one month after nephrectomy, and almost inexistent in healthy donors. This reinforces the specific correlation of SEMA7A to renal cell carcinoma. More importantly, SEMA7A was detected in all four grades of RCC at similar levels, suggesting that it could be an early stage biomarker. Taken altogether, these data define SEMA7A as an early circulating biomarker of renal cell carcinoma, allowing early diagnosis in a non-invasive manner.

Accordingly, in a first aspect, the invention relates to a method for diagnosing Renal cell carcinoma (RCC) in a subject comprising the steps of: i) measuring the expression level of soluble SEMA7A in a fluid sample; ii) comparing the expression measured at step i) with its predetermined reference value, and iii) concluding that the subject suffers from RCC when the expression level of SEMA7A is higher than its predetermined reference value or concluding that the subject does not suffer from RCC when the expression level of SEMA7A is lower than its predetermined reference value.

As used herein, the term "diagnosing" refers to classifying a disease or a symptom, determining a severity of the disease, monitoring disease progression, forecasting an outcome of a disease and/or prospects of recovery.

As used herein, the term "Renal cell carcinoma" also known as renal cell cancer or renal cell adenocarcinoma is the most common type of kidney cancer. Renal cell cancer starts in the lining of the tubules and count different histologic subtypes of renal cancer amongst which the clear cell renal carcinoma (ccRCC, most frequent type with almost 80% of cases), papillary renal cell cancer, chromophobe renal cell cancer and other less common types. A RCC's stage is based upon the size of the tumour (T), the spread of the cancer to the nearby lymph nodes (N) and if there are signs of cancer in other organs such as liver, lung, bone (M). RCC stages range from stage I, meaning the tumour is smaller than 7 cm (approximately 3 inches) and has not spread outside the kidney, to stage IV, meaning the tumour has spread to the outer layers of the kidney or to distant lymph node(s) or other organ(s). In general, lower stage cancers are less aggressive or advanced and less likely to come back after treatment compared with higher stage cancers. Stage I, II, and III RCCs are referred to as localized RCCs, while stage IV is referred to as an advanced or metastatic RCC. In a particular embodiment, the subject is or is susceptible of suffering from early stage of RCC. As used herein, the term "early stage" means that the cancer has not spread beyond the kidney or the axillary lymph nodes. It includes stage I and II as described above.

As used herein, the term "subject" refers to any mammals, such as a rodent, a feline, a canine, and a primate. Particularly, in the present invention, the subject is a human. In a particular embodiment, the subject is a human who is susceptible to have RCC.

As used herein, the term "SEMA7A" refers to semaphorin-7A. This protein has a role in integrin-mediated signaling and functions both in regulating cell migration and immune responses. It promotes formation of focal adhesion complexes, activation of the protein kinase PTK2/FAK1 and subsequent phosphorylation of MAPK1 and MAPK3. The naturally occurring human SEM7A gene has a nucleotide sequence as shown in Genbank Accession number NM_001146029.2, NM_003612.4, NM_001146030.2 and the naturally occurring human SEM7A protein has an aminoacid sequence as shown in Genbank Accession number NP_001139501.1, NP_003603.1, NP_001139502.1. The murine nucleotide and amino acid sequences have also been described (Genbank Accession numbers NM_011352.2 and NP_035482.1). Two isoforms of SEMA7A are known: a soluble form of SEMA7A and a membrane bound form SEMA7A. In the context of the invention, the SEMA7A is a soluble or secreted protein. As used herein, the term "soluble" or "secreted" form refers to a protein, which is actively transported out of the cell while a membrane protein is associated or attached to the membrane of a cell or an organelle inside the cell. In humans, cells such as endocrine cells and B-lymphocytes are specialized in the secretion of proteins, but all cells in the body secrete proteins to a varying degree.

As used herein, the term "fluid sample" refers to blood, saliva, breast milk, urine, semen, blood plasma, synovial fluid, serum, or cerebrospinal fluid, and tissue homogenates. In a particular embodiment, the fluid sample is plasma sample. Plasma refers to a yellow fluid that transports blood cells and platelets around the body and contains a number of substances, including proteins, mineral, salts and hormones. Typically, the blood is mixed with ETDA for example, avoiding the clotting, then centrifuged. A supernatant fluid is obtained which corresponds to the plasma. In a particular embodiment, the fluid sample is urine sample.

As used herein, the term "measuring the expression level" refers to determinate or quantify the expression or presence of a marker in the cell. Methods for determining or measuring the expression of a marker in the cell are well known in the art. The detection and quantification of a marker that is expressed by a cell is performed by flow cytometry. As being also intra cellularly located, SEMA7A expression may be assessed by intracellular flow cytometry using a labeled anti SEMA7A antibodies. Intracellular flow cytometry typically involves the permeabilization and fixation of the cells (e.g. T cells). Any convenient means of permeabilizing and fixing the cells may be used in practicing the methods. For example permeabilizing agent typically include saponin, methanol, Tween® 20, Triton X-100TM. In a particular embodiment, intracellular expression level of SEMA7A is performed by immunological detection. Typically, the immunological detection or quantification of the SEMA7A expression level is achieved by any methods known in the art using at least one antibody that binds specifically to SEMA7A. Examples of said methods include, but are not limited to, standard electrophoretic and immunodiagnostic techniques such as western blots, immuno -precipitation assay, radioimmunoassay, ELISA (enzyme- linked immunosorbent assay), "sandwich" immunoassay, gel diffusion precipitation reaction, immunodiffusion assay, precipitation reaction, agglutination assay (such as gel agglutination assay, hemagglutination assay, etc.), complement fixation assay, protein A assay, immunoelectrophoresis assay, high performance liquid chromatography, size exclusion chromatography, solid-phase affinity, etc. In a particular embodiment, the expression level of SEMA7A is measured by ELISA.

As used herein, the term "predetermined reference value" refers to a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. For example, retrospective measurement in properly banked historical subject samples may be used in establishing the predetermined reference value. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. For example, after determining the expression level of the selected peptide in a group of reference, one can use algorithmic analysis for the statistic treatment of the expression levels determined in samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is high. This algorithmic method is preferably done with a computer. Existing software or systems in the art may be used for the drawing of the ROC curve, such as: MedCalc 9.2.0.1 medical statistical software, SPSS 9.0, ROCPOWER.SAS, DESIGNROC.FOR, MULTIREADER POWER.SAS, CREATE-ROC.SAS, GB STAT VI0.0 (Dynamic Microsystems, Inc. Silver Spring, Md., USA), etc.

### Method of treating RCC

The subject as diagnosed with the method of the invention can be treated with an inhibitor of SEMA7A.

Accordingly, in a second aspect, the invention relates to an inhibitor of SEMA7A for use in the treatment of RCC in a subject in need thereof.

More particularly, the medical use according to the invention comprising: i) determining-whether a subject suffers from early stages of RCC according to and ii) administrating to said subject a therapeutically amount of inhibitor of SEMA7A when the expression level of SEMA7A is higher than its predetermined reference value.

As used herein, the terms "treating" or "treatment" refer to both prophylactic or preventive treatment as well as curative or disease modifying treatment, including treatment of subject at risk of contracting the disease or suspected to have contracted the disease as well as subject who is ill or have been diagnosed as suffering from a disease or medical condition, and includes suppression of clinical relapse. The treatment may be administered to a subject having a medical disorder or who ultimately may acquire the disorder, in order to prevent, curc, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

As used herein, the term "inhibitor of SEMA7A" refers to a natural or synthetic compound that has a biological effect to inhibit the activity or the expression of SEMA7A. Such inhibitor inhibits growth of RCC cell that overexpresses SEMA7A.

In a particular embodiment, the inhibitor of SEMA7A is a peptide, petptidomimetic, small organic molecule, antibody, aptamers, siRNA or antisense oligonucleotide. The term "peptidomimetic" refers to a small protein-like chain designed to mimic a peptide. In a particular embodiment, the inhibitor of inhibitor of SEMA7A is an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity.

In a particular embodiment, the inhibitor of inhibitor of SEMA7A is a small organic molecule. The term "small organic molecule" refers to a molecule of a size comparable to those organic molecules generally used in pharmaceuticals. The term excludes biological macromolecules (e.g., proteins, nucleic acids, etc.). Preferred small organic molecules range in size up to about 5000 Da, more preferably up to 2000 Da, and most preferably up to about 1000 Da.

In some embodiments, the inhibitor of SEMA7A is an antibody. As used herein, the term "antibody" is used in the broadest sense and specifically covers monoclonal antibodies, polyclonal antibodies, multispecific antibodies (e.g. bispecific antibodies) formed from at least two intact antibodies, and antibody fragments so long as they exhibit the desired biological activity. The term includes antibody fragments that comprise an antigen binding domain such as Fab', Fab, F(ab')2, single domain antibodies (DABs), TandAbs dimer, Fv, scFv (single chain Fv), dsFv, ds-scFv, Fd, linear antibodies, minibodies, diabodies, bispecific antibody fragments, bibody, tribody (scFv-Fab fusions, bispecific or trispecific, respectively); sc-diabody; kappa(lamda) bodies (scFv-CL fusions); BiTE (Bispecific T-cell Engager, scFv-scFv tandems to attract T cells); DVD-Ig (dual variable domain antibody, bispecific format); SIP (small immunoprotein, a kind of minibody); SMIP ("small modular immunopharmaceutical" scFv-Fc dimer; DART (ds-stabilized diabody "Dual Affinity ReTargeting"); small antibody mimetics comprising one or more CDRs and the like. The techniques for preparing and using various antibody-based constructs and fragments are well known in the art (see Kabat et al., 1991). Diabodies, in particular, are further described in EP 404, 097 and WO 93/1 1 161; whereas linear antibodies are further described in Zapata et al. (1995). Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, Fv, dsFv, Fd, dAbs, TandAbs, ds-scFv, dimers, minibodies, diabodies, bispecific antibody fragments and other fragments can also be synthesized by recombinant techniques or can be chemically synthesized. Techniques for producing antibody fragments are well known and described in the art. For example, each of Beckman et al., 2006; Holliger & Hudson, 2005; Le Gall et al., 2004; Reff & Heard, 2001 ; Reiter et al., 1996; and Young et al., 1995 further describe and enable the production of effective antibody fragments. In some embodiments, the antibody is a "chimeric" antibody as described in U.S. Pat. No. 4,816,567. In some embodiments, the antibody is a humanized antibody, such as described U.S. Pat. Nos. 6,982,321 and 7,087,409. In some embodiments, the antibody is a human antibody. A "human antibody" such as described in US 6,075,181 and 6,150,584. In some embodiments, the antibody is a single domain antibody such as described in EP 0 368 684, WO 06/030220 and WO 06/003388. In a particular embodiment, the inhibitor is a monoclonal antibody. Monoclonal antibodies can be prepared and isolated using any technique that provides for the production of antibody molecules by continuous cell lines in culture. Techniques for production and isolation include but are not limited to the hybridoma technique, the human B-cell hybridoma technique and the EBV-hybridoma technique.

In a particular, the inhibitor of SEMA7A is an intrabody having specificity for SEMA7A. As used herein, the term "intrabody" generally refer to an intracellular antibody or antibody fragment. Antibodies, in particular single chain variable antibody fragments (scFv), can be modified for intracellular localization. Such modification may entail for example, the fusion to a stable intracellular protein, such as, e.g., maltose binding protein, or the addition of intracellular trafficking/localization peptide sequences, such as, e.g., the endoplasmic reticulum retention. In some embodiments, the intrabody is a single domain antibody. In some embodiments, the antibody according to the invention is a single domain antibody. The term "single domain antibody" (sdAb) or "VHH" refers to the single heavy chain variable domain of antibodies of the type that can be found in Camelid mammals which are naturally devoid of light chains. Such VHH are also called "nanobody®". According to the invention, sdAb can particularly be llama sdAb.

In some embodiments, the inhibitor of SEMA7A is a short hairpin RNA (shRNA), a small interfering RNA (siRNA) or an antisense oligonucleotide which inhibits the expression of SEMA7A. In a particular embodiment, the inhibitor of SEMA7A 4 expression is siRNA. A short hairpin RNA (shRNA) is a sequence of RNA that makes a tight hairpin turn that can be used to silence gene expression via RNA interference. shRNA is generally expressed using a vector introduced into cells, wherein the vector utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs that match the siRNA to which it is bound. Small interfering RNA (siRNA), sometimes known as short interfering RNA or silencing RNA, are a class of 20-25 nucleotide-long double- stranded RNA molecules that play a variety of roles in biology. Most notably, siRNA is involved in the RNA interference (RNAi) pathway whereby the siRNA interferes with the expression of a specific gene. Anti-sense oligonucleotides include anti-sense RNA molecules and anti-sense DNA molecules, would act to directly block the translation of the targeted mRNA by binding thereto and thus preventing protein translation or increasing mRNA degradation, thus decreasing the level of the targeted protein, and thus activity, in a cell. For example, antisense oligonucleotides of at least about 15 bases and complementary to unique regions of the mRNA transcript sequence can be synthesized, e.g., by conventional phosphodiester techniques. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732). Antisense oligonucleotides, siRNAs, shRNAs of the invention may be delivered in vivo alone or in association with a vector. In its broadest sense, a "vector" is any vehicle capable of facilitating the transfer of the antisense oligonucleotide, siRNA, shRNA or ribozyme nucleic acid to the cells and typically mast cells. Typically, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the antisense oligonucleotide, siRNA, shRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

In some embodiments, the inhibitor of SEMA7A expression is an endonuclease. In the last few years, staggering advances in sequencing technologies have provided an unprecedentedly detailed overview of the multiple genetic aberrations in cancer. By considerably expanding the list of new potential oncogenes and tumor suppressor genes, these new data strongly emphasize the need of fast and reliable strategies to characterize the normal and pathological function of these genes and assess their role, in particular as driving factors during oncogenesis. As an alternative to more conventional approaches, such as cDNA overexpression or downregulation by RNA interference, the new technologies provide the means to recreate the actual mutations observed in cancer through direct manipulation of the genome. Indeed, natural and engineered nuclease enzymes have attracted considerable attention in the recent years. The mechanism behind endonuclease-based genome inactivating generally requires a first step of DNA single or double strand break, which can then trigger two distinct cellular mechanisms for DNA repair, which can be exploited for DNA inactivating: the errorprone nonhomologous end-joining (NHEJ) and the high-fidelity homology-directed repair (HDR).

In a particular embodiment, the endonuclease is CRISPR-cas. As used herein, the term "CRISPR-cas" has its general meaning in the art and refers to clustered regularly interspaced short palindromic repeats associated which are the segments of prokaryotic DNA containing short repetitions of base sequences.

In some embodiment, the endonuclease is CRISPR-cas9 which is from Streptococcus pyogenes. The CRISPR/Cas9 system has been described in US 8697359 B1 and US 2014/0068797. Originally an adaptive immune system in prokaryotes (Barrangou and Marraffini, 2014), CRISPR has been recently engineered into a new powerful tool for genome editing. It has already been successfully used to target important genes in many cell lines and organisms, including human (Mali et al., 2013, Science, Vol. 339 : 823-826), bacteria (Fabre et al., 2014, PLoS Negl. Trop. Dis., Vol. 8:e2671.), zebrafish (Hwang et al., 2013, PLoS One, Vol. 8:e68708.), C. elegans (Hai et al., 2014 Cell Res. doi: 10.1038/cr.2014.11.), bacteria (Fabre et al., 2014, PLoS Negl. Trop. Dis., Vol. 8:e2671.), plants (Mali et al., 2013, Science, Vol. 339 : 823-826), Xenopus tropicalis (Guo et al., 2014, Development, Vol. 141 : 707-714.), yeast (DiCarlo et al., 2013, Nucleic Acids Res., Vol. 41 : 4336-4343.), Drosophila (Gratz et al., 2014 Genetics, doi:10.1534/genetics.113.160713), monkeys (Niu et al., 2014, Cell, Vol. 156 : 836-843.), rabbits (Yang et al., 2014, J. Mol. Cell Biol., Vol. 6 : 97-99.), pigs (Hai et al., 2014, Cell Res. doi: 10.1038/cr.2014.11.), rats (Ma et al., 2014, Cell Res., Vol. 24 : 122-125.) and mice (Mashiko et al., 2014, Dev. Growth Differ. Vol. 56 : 122-129.). Several groups have now taken advantage of this method to introduce single point mutations (deletions or insertions) in a particular target gene, via a single gRNA. Using a pair of gRNA-directed Cas9 nucleases instead, it is also possible to induce large deletions or genomic rearrangements, such as inversions or translocations. A recent exciting development is the use of the dCas9 version of the CRISPR/Cas9 system to target protein domains for transcriptional regulation, epigenetic modification, and microscopic visualization of specific genome loci.

In some embodiment, the endonuclease is CRISPR-Cpfl which is the more recently characterized CRISPR from Provotella and Francisella 1 (Cpf1) in Zetsche et al. ("Cpfl is a Single RNA-guided Endonuclease of a Class 2 CRISPR-Cas System (2015); Cell; 163, 1-13).

The subject as diagnosed according to the invention, can also be treated with anti-angiogenic or anti-mTOR drugs. Also disclosed is a method of treating RCC in a subject in need thereof comprising a step of administrating to said subject a therapeutically amount of anti-angiogenic drugs.

In a particular example, the disclosure relates to a method of treating RCC in a subject in need thereof comprising the steps of: i) determining whether a subject suffers from of RCC according to method as described above and ii) administering to said subject a therapeutically amount of anti-angiogenic drugs when the expression level of SEMA7A is higher than its predetermined reference value.

In a particular example, the subject suffers or is susceptible of suffering from early stage of RCC.

As used herein, the term "anti-angiogenic drug" also called as angiogenesis inhibitor refers to a substance that inhibits the growth of blood vessels (angiogenesis). In the context of the invention, the tumour (e.g RCC) can stimulate the growth of new vessels to expand the tumour to other organs. Thus, inhibiting angiogenesis is needed to reduce or stop the growth of tumours. Inhibiting angiogenesis requires treatment with anti-angiogenic factors, or drugs which reduce the production of pro-angiogenic factors, preventing receptor binding or blocking their actions. The pro angiogenic factor well studied and known is vascular endothelial growth factor (VEGF). The VEGF family comprises in mammals five members: VEGF-A, placenta growth factor (PGF), VEGF-B, VEGF-C and VEGF-D. The blood vessels feeding RCC tumours are particularly dependent on VEGF, so treatment with anti-VEGF therapies can damage tumour blood vessels, and this may slow or stop the tumour from growing for long periods of time. At least one of the inhibitors of VEGF is selected from the group consisting of: Pazopanib (brand name: Votrient), Sunitinib (brand name: Sutent), Cabozantinib (brand name: Cabometyx), Axitinib (brand name: Inlyta), Sorafenib (brand name: Nexavar) and Bevacizumab (brand name: Avastin).

Pazopanib is a selective multi-targeted receptor tyrosine kinase inhibitor that blocks tumour growth and inhibits angiogenesis. It has the following formula C21H23N7O2S and structure in the art:

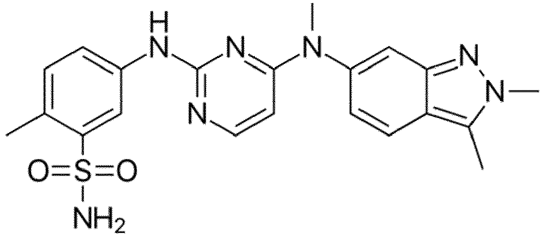

Sunitinib is a selective multi-targeted receptor tyrosine kinase inhibitor that blocks tumour growth and inhibits angiogenesis. It has the following formula C22H27FN4O2 and structure in the art:

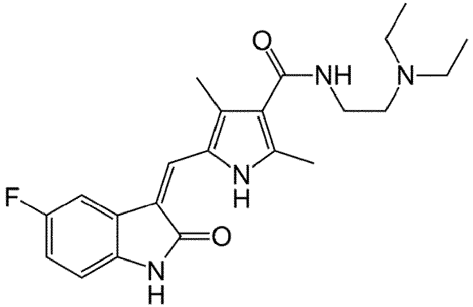

Cabozantinib is a selective multi-targeted receptor tyrosine kinase inhibitor that blocks tumour growth and inhibits angiogenesis. It has the following formula C28H24FN3O5 and structure in the art:

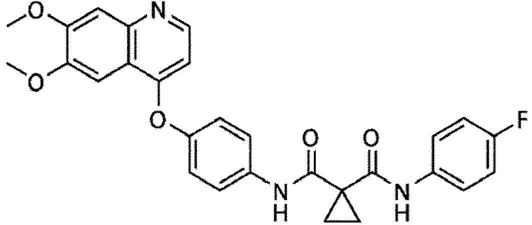

Axitinib is a selective multi-targeted receptor tyrosine kinase inhibitor that blocks tumour growth and inhibits angiogenesis. It has the following formula C22H18N4OS and structure in the art:

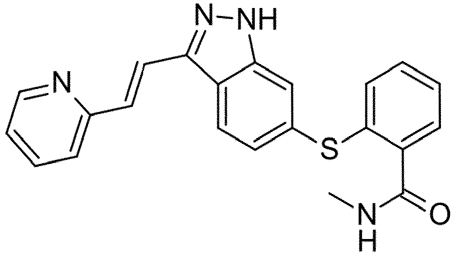

Sorafenib is a selective multi-targeted receptor tyrosine kinase inhibitor that blocks tumour growth and inhibits angiogenesis. It has the following formula C21H16ClF3N4O3 and structure in the art:

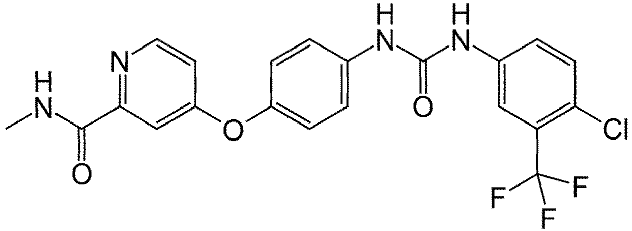

Bevacizumab is a recombinant humanized monoclonal antibody that blocks angiogenesis by inhibiting vascular endothelial growth factor A (VEGFA). It has the following protein chemical formula in the art: C6538H10034N1716O2033S44 and has the accession number drug bank: DB00112 (BTD00087, BIOD00087).

Disclosed is a method of treating RCC in a subject in need thereof comprising a step of administrating to said subject a therapeutically amount of anti-mTOR drugs. Disclosed is a method of treating RCC in a subject in need thereof comprising the steps of: i) determining whether a subject suffers from RCC according to the method as described above and ii) administering to said subject a therapeutically amount of anti-mTOR drugs when the expression level of SEMA7A is higher than its predetermined reference value.

In a particular example, the subject suffers or is susceptible of suffering from early stage of RCC.

As used herein, the term "anti-mTOR drugs" or "mTOR inhibitors" refer to a substance that blocks mTOR. As used herein the term "mTOR" refers to mechanistic (or mammalian) target of rapamycin. mTOR also known as FK506 binding protein 12-rapamycin associated protein 1 (FRAP1), is a protein which in humans is encoded by the FRAP1 gene. mTOR is a serine/threonine protein kinase (and specifically belongs to the phosphatidylinositol 3-kinase-related kinase protein family) that regulates cell growth, cell proliferation, cell motility, cell survival, protein synthesis and transcription. mTOR is the catalytic subunit of the two molecular complexes mTORCI and mTORC2. The naturally occurring human mTOR gene has a nucleotide sequence as shown in Genbank Accession number NM_004958 and the naturally occurring human mTOR protein has an aminoacid sequence as shown in Genbank Accession number NP_004949.1. The murine nucleotide and amino acid sequences have also been described (Genbank Accession numbers NM_020009 and NP_064393.2). mTORC1 inhibitors are well-known in the art as illustrated by Mohindra et al., 2014; Nelson et al., 2013; Pal and Quinn, 2013. In one embodiment of the invention, mTORC1 inhibitors include but are not limited to Rapamycin, temsirolimus (brand name: Torisel; 42-[2,2-bis (hydroxymethyl)] rapamycin, also known as CCI779), everolimus (brand name: Afinitor; 42-O-(2-hydroxyethyl) rapamycin, also known as RAD001), and ridaforolimus (macrolide dimethylphophinic acid rapamycin-40-O-yl ester derivative of sirolimus, also known as AP23573 and deforolimus) (Mohindra et al., 2014; Nelson et al., 2013; Pal and Quinn, 2013). The term "rapamycin" also known as sirolimus has its general meaning in the art and relates to a naturally occurring product that was isolated and purified from the soil bacteria Streptomyces hygroscopicus. Rapamycin was initially utilized for its antifungal and immunosuppressive properties before its antiproliferative properties were clinically appreciated. When the role of mTOR dysregulation became more evident in tumourigenesis, rapamycin was re-evaluated for an antineoplastic role. Despite success in preclinical models, rapamycin was difficult to develop into an anticancer drug due to problems with stability and pharmacokinetics. Semisynthetic analogs to rapamycin, known as rapalogs (first-generation mTOR inhibitors), were later developed with improved pharmacokinetics and easier drug delivery. The rapalogs include temsirolimus (CCI779), everolimus (RAD001), and ridaforolimus (AP23573, also known as deforolimus). Rapamycin and the rapalogs work by binding an intracellular protein, FKBP-12, and forming a complex that allosterically inhibits the FRB domain of mTORC1. The rapalogs have no direct effect on mTORC2, although there have been some reports indicating that some cell lines show mTORC2 inhibition after prolonged exposure to rapamycin (Mohindra et al., 2014; Nelson et al., 2013). As used herein, the term "rapamycin" and "rapamycin derivatives" or "rapalogs" has its general meaning in the art and refers to compounds described in Mohindra et al., 2014; Nelson et al., 2013; Pal and Quinn, 2013 having the general formula (I):

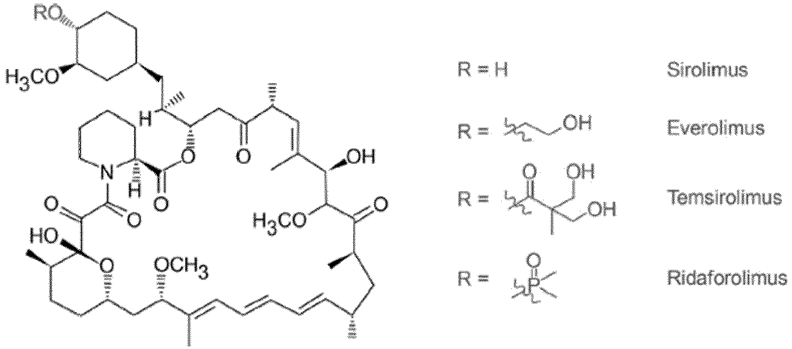

The term "rapamycin derivatives" also refers to compounds described in WO 94/09010, WO 95/16691, WO 96/41807 and US 2014/0105895.

### Method of predicting the risk of having a relapse

In many cases, the subjects suffering from RCC are taken in charge very late with the disease being already well spread, and very often at metastatic stage, with poor prognosis. Indeed, most people with RCC show symptoms such as hematuria, pain in the flank, a mass in the abdomen together with an important weight loss and fever. However, most of these symptoms are not exclusive to RCC and do not occur until advanced stages of the disease. RCC develops asymptomatically for a long period of time and is often diagnosed incidentally. The only option to treat patients with metastatic stage RCC remains partial or total nephrectomy. Accordingly, the biomarker of the invention is suitable to predict the risk of relapse in a subject after a partial or total nephrectomy.

Accordingly, in a fourth aspect, the invention relates to a method of predicting the risk of having a relapse in a subject after a partial or total nephrectomy comprising the steps of: i) measuring the expression level of soluble SEMA7A in a fluid sample, ii) comparing the expression measured at step i) with its predetermined reference value, and iii) concluding that the subject has a risk of relapse when the expression level of SEMA7A is higher than its predetermined reference value or concluding that the subject has not a risk of relapse when the expression level of SEMA7A is lower than its predetermined reference value.

As used herein the term "predicting" means that the subject to be analyzed by the method of the invention is allocated either into the group of subjects who will relapse, or into a group of subjects who will not relapse after a partial or total nephrectomy.

As used herein, the term "risk" in the context of the present invention, relates to the probability that an event will occur over a specific time period, as in the conversion to relapse, and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a subject compared either to the absolute risks of low risk cohorts or an average population risk, which can vary by how clinical risk factors are assessed. Odds ratios, the proportion of positive events to negative events for a given test result, are also commonly used (odds are according to the formula p/(l-p) where p is the probability of event and (1- p) is the probability of no event) to no- conversion. "Risk evaluation," or "evaluation of risk" in the context of the present invention encompasses making a prediction of the probability, odds, or likelihood that an event or disease state may occur, the rate of occurrence of the event or conversion from one disease state to another, i.e., from a normal condition to relapse or to one at risk of developing relapse. Risk evaluation can also comprise prediction of future clinical parameters, traditional laboratory risk factor values, or other indices of relapse, either in absolute or relative terms in reference to a previously measured population. The methods of the present invention may be used to make continuous or categorical measurements of the risk of conversion to relapse, thus diagnosing and defining the risk spectrum of a category of subjects defined as being at risk of having relapse. In the categorical scenario, the invention can be used to discriminate between normal and other subject cohorts at higher risk of having relapse. In some embodiments, the present invention may be used so as to discriminate those at risk of having relapse from normal, or those having relapse disease from normal.

As used herein, the terms "relapse" or "cancer recurrence" refer to the return of signs and symptoms of a disease (e.g. RCC) after a subject has enjoyed a remission after a treatment. Thus, if initially the target disease is alleviated or healed, or progression of the disease was halted or slowed down, and subsequently the disease or one or more characteristics of the disease return, the subject is referred to as being "relapsed."

As used herein, the term "subject" refers to any mammals, such as a rodent, a feline, a canine, and a primate. Particularly, in the present invention, the subject is a human. In a particular embodiment, the subject is a human who has a partial or total nephrectomy.

As used herein, the "a partial or total nephrectomy" refers to the surgical removal of a kidney partially or totally. Typically, partial nephrectomy is the surgical removal of a kidney tumour along with a thin rim of normal kidney, with the two aims of curing the cancer and preserving as much normal kidney as possible. During a complete (total or radical) nephrectomy, the surgeon removes the entire kidney.

When a subject is identified as having a risk of relapse after a partial or total nephrectomy, the physician could administer to said subject a therapeutically amount of anti-angiogeneic drugs or anti-mTOR drugs. In the context of the total nephrectomy, the physician can decide whether the subject should be transplanted.

As used herein, the term "transplanted" also called "grafted" subject, refers to a subject who has received an organ transplantation. The term "organ transplantation" refers to the procedure of replacing diseased organs, parts of organs, or tissues by healthy organs or tissues. The transplanted organ or tissue can be obtained either from the subject himself (= autograft), from another human donor (= allograft) or from an animal (= xenograft). Transplanted organs may be artificial or natural, whole (such as kidney, heart, lung and liver) or partial (such as heart valves, lung, skin and bone). In a particular embodiment, the subject may be kidney transplanted.

### Kit for performing the method according to the invention

In a fifth aspect, the invention relates to the use of a kit for performing the methods of the present invention, wherein said kit comprises means for measuring the expression level of soluble SEMA7A in a fluid sample. More particularly, the kit used in the methods comprising:
(a) at least one reagent for measuring the expression of SEMA7A in a fluid sample obtained from a subject, wherein when the expression level of soluble SEMA7A is higher than its predetermined reference, it is indicative that the subject suffers from RCC;
(b) at least one control; and
(c) instructions for use.

The at least one reagent can be, for example, a SEMA7A-specific antibody, a pair of Sema7A-specific primers, etc. Typically, the kit used in the methods includes at least one reagent for detecting the expression level of soluble SEMA7A (e.g., detecting SEMA7A DNA and mRNA levels, detecting SEMA7A protein levels) in a fluid sample (e.g., blood sample, etc) from the subject, at least one control, and instructions for use.

In one embodiment, a kit used in the methods includes a monoclonal or polyclonal antibody to SEMA7A 7A, a detectable label, and instructions for use.

In another embodiment, the kit used in the methods includes SEMA7A -specific primers for PCR, e.g., PCR and RT-PCR, and instructions for use. The controls for RNA and DNA can be housekeeping genes such as, for example, b-Actin or GAPDH. For protein determination using ELISA kits all the components necessary are contained in the kit. The controls for these are typically samples from non-tumor fluid. Such kits can also be used, for example, to measure a subject's response after a partial or total nephrectomy.

The invention will be further illustrated by the following figures and examples. However, these examples and figures should not be interpreted in any way as limiting the scope of the present invention, which is defined by the appended claims.

### FIGURES:

**Figure 1****: Renal cancer cells secrete semaphorin 7A.** ELISA analysis of secreted semaphorin-7A in conditioned medium collected from renal CSC, renal cancer cells (ACHN) or renal normal epithelial cells (HK2). ** p value < 0.005, * p value < 0.05.
**Figure 2****: Detection of semaphorin 7A in patients with RCC plasmas.** Graph illustrates semaphorin-7A rates in plasmas of patients with RCC versus plasmas of healthy donors. ** p Value = 0.008
**Figure 3****: Semaphorin-7A is detected in Patients with RCC independently of the ontogeny of the tumour.** Histograms represent semaphorin-7A levels found in plasma of patients with localized RCC (A), metastatic RCC (B), or other types of RCC (C) prior any chirurgical intervention.

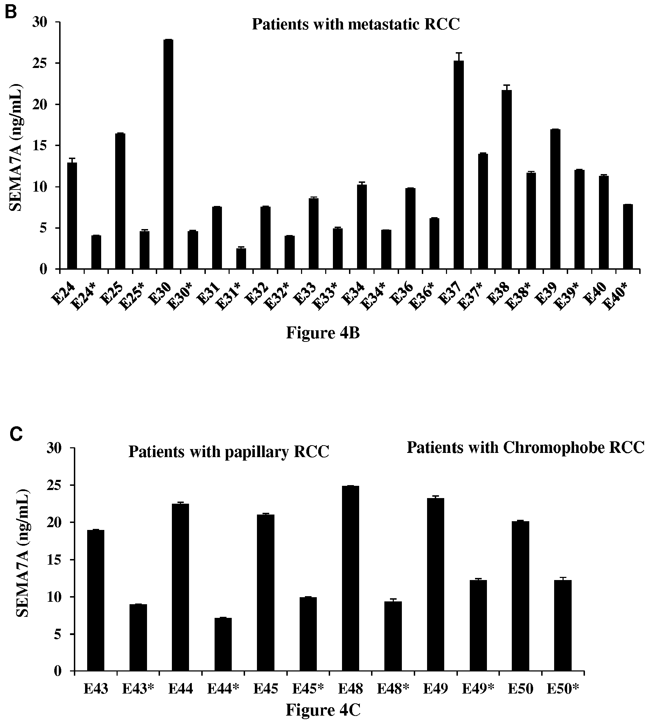

**Figure 4****: Semaphorin-7A rates are lower in Patients with RCC 1 month after surgery.** Histograms depict semaphorin-7A levels found in plasma of patients with localized RCC (A), metastatic RCC (B), or other types of RCC (C) before (black histograms) or 1 month post-surgery. * corresponds to the 1-month post-surgery sample of each patient.
**Figure 5****: Semaphorin-7A is detected in all four grades of renal cancer.** Graph illustrates semaphorin-7A rates in plasmas of patients with RCC from different ontogeny. Grades I, II, III and IV correspond to the Fuhrman grade of the tumour.
**Figure 6****: SEMA7A levels in patients with early stage renal cell carcinoma.** Graph illustrates semaphorin-7A rates in plasmas of patients with low stages RCC (pTNM 1a, pTNM 1b and 2a) versus plasmas of healthy donors. ** p Value = 0.0052, * p Value = 0.035, ns = non-significant difference.
**Figure 7****: SEMA7A levels in patients with pTNM 1a renal cell carcinoma before and after surgical resection of the tumor.** Histograms depict semaphorin-7A levels found in plasma of patients with low stages RCC (pTNM 1a) before (black histograms) or 1-month post surgery (grey histograms).

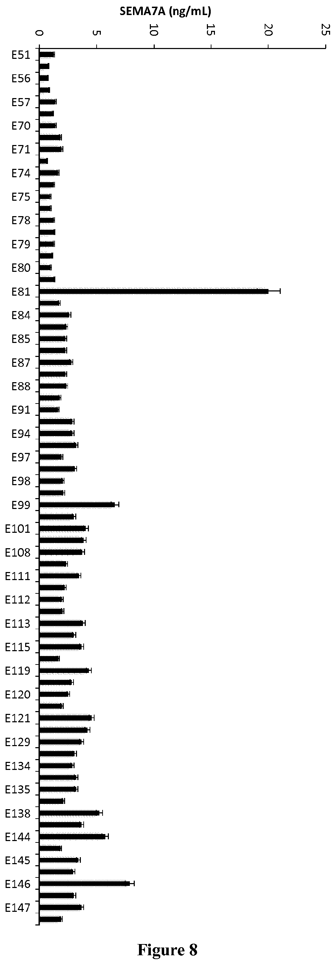

**Figure 8****: SEMA7A levels in patients with pTNM 1b and 2a renal cell carcinoma before and after surgical resection of the tumor.** Histograms depict semaphorin-7A levels found in plasma of patients with low stages RCC (pTNM 1b and 2a) before (black histograms) or 1-month post-surgery (grey histograms).

### EXAMPLES:

### Example 1

### Material & Methods

**Conditioned media preparation.** Renal normal (HK2) and neoplastic cells (CSC and ACHN) were seeded at 10⁶ in a T75 flask and left to grow for 48 hours in their original culture medium (Azzi S et al., 2011). Cells were then cultured for another 48 hours in 3 mL of serum free, additive free medium, and supernatant collected. Samples were then cleared by centrifugation and stored at -20°C.

**Sample preparation, gel migration and proteins processing for mass spectrometry.** Twenty mL of conditioned media were collected from renal cancer stem-like cells, concentrated by ultrafiltration on a 3KDa cutoff Amicon Ultra filters, and then subjected to TCEP (tris (2-carboxyethyl)phosphine) reduction (25 mM Tris, ImM TCEP-HCl, pH 8.0, 10 min at 37°C). Samples were next alkylated with 5 mM iodoacetamide IAA (15 min at 37°C in the dark). Proteins were next precipitated with acetone overnight at -20°C and pellets dried under vacuum. Proteins migration was performed using pre-cast 1mm 10 wells 4-12% NuPAGE Bis-Tris Protein Gels (Pierce, Invitrogen). The gel was washed twice with qH2O, and bands excised using a clean razor blade and tweezers. Slices were transferred to 1.5 ml eppendorf tubes containing 600 µl of a 1:1 (v:v) solution of 100 mM NH4HCO3 / acetonitrile. Proteins were digested with trypsin (12.5 ng/µl) overnight at 37°C. For peptides extraction from the gel prior to mass spectrometry analysis, 50 µl of a 0.5 % formic acid solution (HCOOH) in 30% acetonitrile (ACN) were added, samples sonicated for 2 min and then incubated for 20 min at room temperature. Supernatants were next transferred to a low binding eppendorf tube, and peptides extracted by adding 50 µl of ACN/H2O/HCOOH (60/35/5 volume) to gel pieces for 15 minutes at 25°C. This step is repeated by adding 50 µl of ACN/H2O/HCOOH (70/29/1 volume). Pooled supernatants were next dried under vacuum in a speedvac to approximately 10 µl final volume. Extracted peptides were solubilized in 20 µl of ACN/H2O/HCOOH (4.5/95/0.5 volume), spun down by centrifugation for 5 min at 10000g and supernatants transferred to mass spectrometry injection tubes. Mass spectrometric analyses were performed using nanoflow liquid chromatography tandem mass spectrometry (LC-MS/MS). LC-MS/MS was carried out on an RSLC Ultimate-3000 nanoflow LC system (ThermoScientific) coupled to a Hybrid LTQ-Orbitrap Velos mass spectrometer instrument (ThermoScientific).

**Blood samples handling and plasmas preparation.** For patients enrolled at the Kremlin Bicêtre Hospital (Le Kremlin Bicêtre, France), the physician collected blood in EDTA-sampling tubes. Blood were then transferred to a 15 mL Falcon Tube and centrifuged for 15 min at 2000 rpm. Plasma was then collected and stored at -80°C.

Samples obtained from the plasma library were handled and stored by the staff of the CHU Bordeaux (UroCCR, Bordeaux, France). Healthy donors blood was obtained from Saint Louis hospital (Paris, France). Samples are labeled as E and E* corresponding to blood collection before and 30 days post surgery respectively. All patients signed a consent form.

**ELISA Assay**. Semaphorin-7A secreted concentrations were analyzed by ELISA assay in cells-conditioned (CM) medium and patient's plasmas. Briefly, 50 µl of CM or plasma were coated in a 96 well ELISA plate at 4°C overnight, and saturated (BSA 3%, 30 min). Primary antibody against semaphorin-7A was then added to each well and the mixture was incubated for 2 hours at room temperature. Wells were washed 5 times with the washing solution (PBS IX + 0.1% Tween 20) and further stained with an anti-mouse HRP-conjugated IgG for 1 hour at room temperature (Jackson ImmunoResearch antibodies). Plates were washed five times, and 50 µl of TMB (3,3',5,5'-tetramethylbenzidine) substrate (Sigma Aldrich) was added for 20 min in the dark. Absorbance was monitored at 650 nm. Recombinant Semaphorin-7A (R&D systems) was used to establish the standard curve.

| | Accession | Description | Score | Coverage # | Proteins # | Unique peptides # | Peptides # |
|---|---|---|---|---|---|---|---|
| CSC #1 | 075326 | Semaphorin-7A | 21,03 | 5,86 | 2 | 2 | 4 |
| CSC #2 | 075326 | Semaphorin-7A | 51,18 | 22,37 | 3 | 6 | 12 |

Table A: The table summarizes mass spectrometry analysis of conditioned media collected for 2 different renal cancer stem-like cells (CSC). Semaphorin 7A was found secreted by both CSC with a score of 21,03 and 51,18.

### Results

### Renal cancer cells secrete Semaphorin 7A

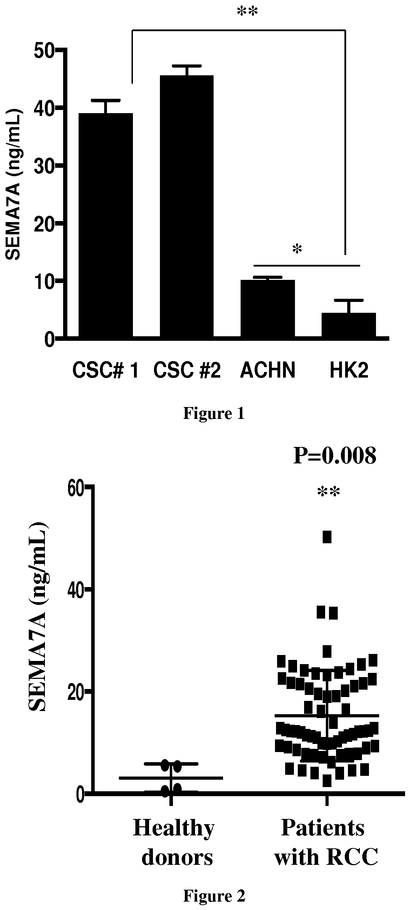

We performed mass spectrometry secretome analysis of two different patients-derived renal cancer stem-like cells (CSC #1 and CSC #2), and found SEMA7A with respective sequence coverage of 5.86 and 22.37 together with 4 and 12 unique peptide sequences (Figure 1A). Consistent with mass spectrometry analysis, ELISA assay (Figure 1B) showed SEMA7A secretion in conditioned media collected from renal CSC as well as renal carcinoma cell line ACHN, 40 ng/mL and 10 ng/mL respectively. Interestingly SEMA7A levels were significantly lower (2-3 ng/mL) in normal renal epithelial cells secretome (HK2), suggesting that SEMA7A secretion is likely a new biomarker of renal cancer cells.

### Semaphorin 7A is detected in the plasma of patients with RCC independently of the tumour ontogeny

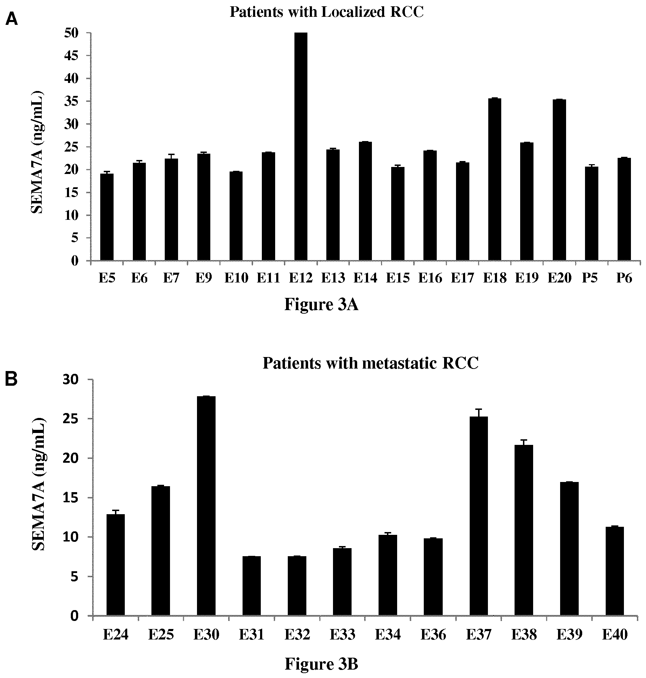

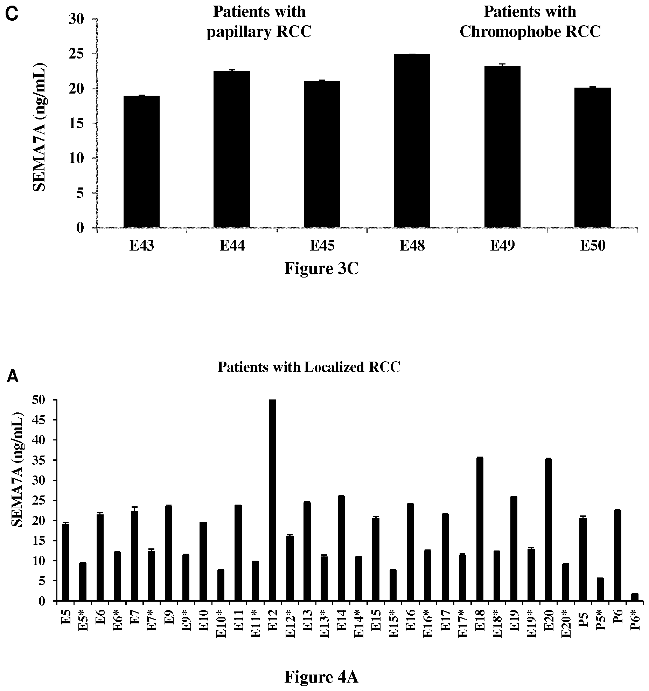

We further performed bioinformatics big data analysis of the GSE15641 Affymetrix transcriptome available data on RCC patient's cells. We found that SEMA7A was overexpressed in patients with RCC when compared to healthy donors. We next asked the question whether SEMA7A could be detected in patients with RCC biologic fluids. For this aim, we performed an ELISA assay comparing plasmas from healthy donors and patients with RCC. We found that SEMA7A was detected in all patients with a mean of 20 ng/mL. Strikingly, healthy donors showed none or very low rates with a mean of 2 ng/mL (Figure 2). We further addressed the potential correlation between SEMA7A levels and the subtypes of RCC. Renal cell carcinoma counts different histologic subtypes such as clear cell renal carcinoma (ccRCC), papillary RCC and chromophobe RCC. ELISA assay analysis shows that SEMA7A secretion was not restricted to a specific subtype. Indeed, the protein could be detected in plasma from patients with localized or metastatic RCC (Figure 3A-B) as well as patients with papillary or chromophobe tumour (Figure 3C). Of note, SEMA7A levels were found slightly higher in patients with localized RCC when compared to patients with metastatic tumour (means of 25ng/mL and 15ng/mL respectively). This could be, in part explained, by the SEMA7A important angiogenic potential. Indeed, the protein was already described as a neovascularization key element in a cornea experimental model (Ghanem R et al. 2011). One could possibly imagine that its levels is particularly higher in localized RCC, in order to promote tumour angiogenesis and therefore allow tumour growth and metastasis.

### Semaphorin 7A levels drop after surgery and are not correlated to tumour

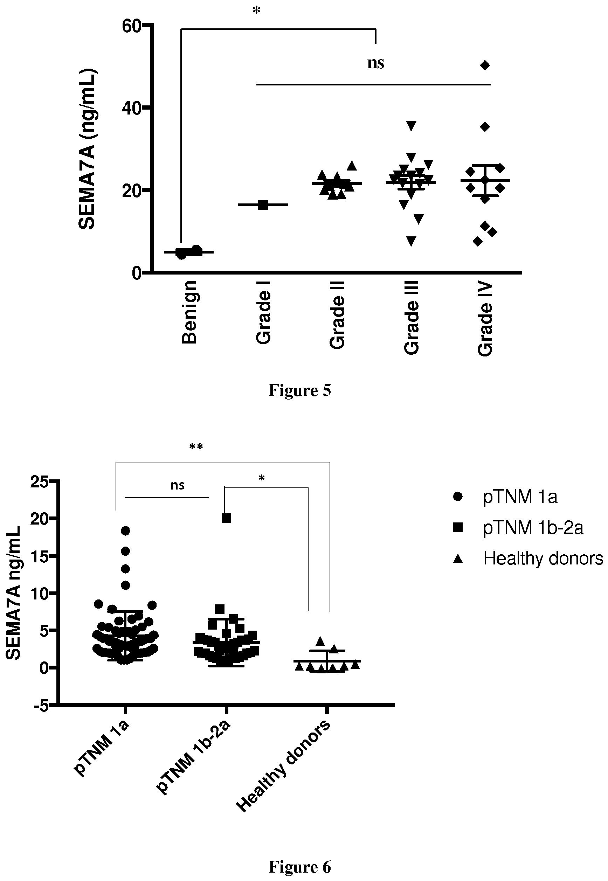

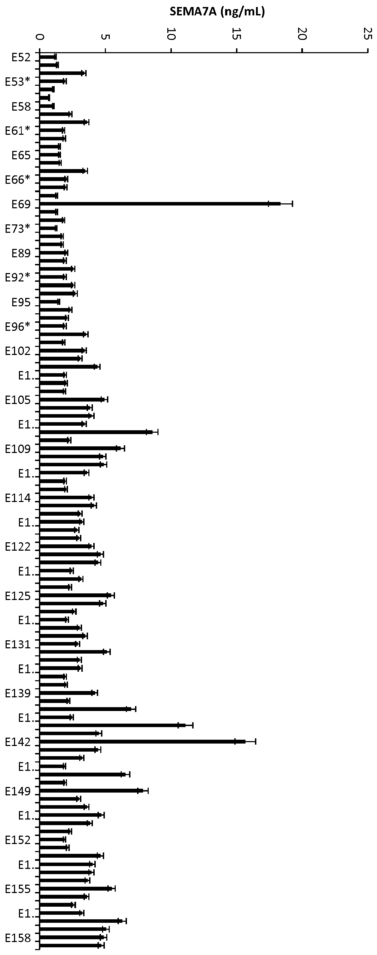

The use of circulating tumour biomarkers is not restricted to disease diagnosis, and is often employed as a monitoring tool to detect cancer recurrence well before any clinical or radiological evidence (Sharma S. 2009). In this context, we evaluated SEMA7A levels in patients with RCC who underwent partial or total nephrectomy. ELISA assay was performed on plasma samples collected from patients before and 30 days post surgical resection of the tumour mass. Interestingly, SEMA7A levels were 3 to 5 times lower after surgery in all patients regardless the ontogeny of RCC. For instance, SEMA7A levels dropped from 25 ng/mL to 7 ng/mL in patients with localized RCC, from 15 ng/mL to 5 ng/mL in patients with metastatic RCC, and from 25 ng/mL to 7 ng/mL in patients with papillary and chromophobe RCC. However, in some patients with metastatic RCC, SEMA7A levels did not drastically drop, going from 17 ng/mL to 12 ng/mL (Patient E39), and from 12 ng/mL to 7 ng/mL (Patient E40). This could be in part explained by the residual metastatic tumour, as surgery only allows the resection of the renal mass. Albeit lower than in our previous results (Figure 3), we still observe a decrease in SEMA7A levels after surgery in almost all patients regardless the ontogeny of RCC (Figure 6 and 7), and this is often represented by 2 to 4 ng/ml level drop.

Renal cell carcinoma is evaluated and classified according to the Führman grade of aggressiveness, with grade I being the less aggressive form and grade IV the more aggressive one. To evaluate a potential correlation between SEMA7A and the tumour grade, we addressed its plasma levels in patients with benign renal tumours as well as in patients with RCC of grade I to IV. Albeit slightly lower in grade I, no significant difference was observed in SEMA7A levels in all four grades of RCC (Figure 5). As expected, benign renal tumours exhibited very low amounts of the protein (3 ng/mL) reinforcing the specificity of semphroin-7A to neoplastic conditions.

### Example 2

### Material & Methods

**Blood samples handling and plasmas preparation.** Samples obtained from the plasma library were handled and stored frozen by the staff of the CHU Bordeaux (UroCCR, Bordeaux, France). Healthy donors blood was obtained from Saint Louis hospital (Paris, France). All patients signed a consent form.

**ELISA Assay**. Semaphorin-7A secreted concentrations were analyzed by ELISA assay in patient's plasmas. Briefly, 50 µl of plasma were coated in a 96 well ELISA plate at 4°C overnight, and saturated (BSA 3%, 30 min). Primary antibody against semaphorin-7A was then added to each well and the mixture was incubated for 2 hours at room temperature. Wells were washed 5 times with the washing solution (PBS IX + 0.1% Tween 20) and further stained with an anti-mouse HRP-conjugated IgG for 1 hour at room temperature (Jackson ImmunoResearch antibodies). Plates were washed five times, and 50 µl of TMB (3,3',5,5'-tetramethylbenzidine) substrate (Sigma) was added for 20 min in the dark. Absorbance was monitored at 650 nm. Recombinant Semaphorin-7A (R&D systems) was used to establish the standard curve.

### Results

### Semaphorin-7A is detected in the plasma of patients with early stages RCC

We have previously shown that SEMA7A was detected in all patients with RCC with a mean of 20 ng/mL independently of tumor ontogeny. In order to consolidate our previous results setting the semaphoring-7A as a circulating biomarker for renal cell carcinoma, we further addressed its levels in patients with early stages RCC. Kidney tumor stages are defined following a TNM system. The TNM system is based on 3 key pieces of information: the size of the main tumor (T) and whether it has grown into nearby areas, spread to nearby lymph nodes (N), mestastsis to other organs (M). Here, we analyse plasma from patients with stages 1 and 2 (T1a: tumor confined to kidney, <4 cm, T1b: tumor confined to kidney, >4 cm but <7 cm, T2a: tumor confined to kidney, >7 cm but not > 10 cm).

ELISA assay analysis shows that SEMA7A was found in the plasma of all patients, regardless the stages (Figure 6). Indeed, the protein could be detected in plasma from patients with TNM 1a, 1b and 2a. Of note, SEMA7A levels were found slightly lower in patients with early stages RCC (Figure 6) when compared to patients with metastatic tumor (Figure 3) (means of 8ng/mL and 25ng/mL respectively). As expected, plasma from healthy donors exhibited very low amounts of the protein (1-2 ng/mL) reinforcing the specificity of semphroin-7A to neoplastic conditions.

### Conclusions

Taken together, our data report the correlation of SEMA7A levels to renal carcinoma independently of tumour origin and grade, and thus place this protein as a promising and relevant biomarker for diagnosis and monitoring of kidney tumours.

### REFERENCES:

Throughout this application, various references describe the state of the art to which this invention pertains. The disclosures of these references are hereby incorporated by reference into the present disclosure.
Renal cell carcinoma. Paul Cairns, PhD ; Cancer Biomark. 2011 ; 9(1-6): 461-473. doi:10.3233/CBM-2011-0176.
Renal cell carcinoma: a critical analysis of metabolomic biomarkers emerging from current model systems. Daniela Rodrigues, Transl Res. 2016 Aug 2. pii: S1931-5244(16)30152-9. doi: 10.1016/j.trsl.2016.07.018.
The 2016 WHO Classification of Tumours of the Urinary System and Male Genital Organs-Part A: Renal, Penile, and Testicular Tumours. Moch H; Eur Urol. 2016 Jul;70(1):93-105. doi: 10.1016/j.eururo.2016.02.029. Epub 2016 Feb 28.
Renal cell carcinoma 2005: new frontiers in staging, prognostication and targeted molecular therapy. Lam JS; J Urol. 2005 Jun;173(6):1853-62.
Clinical risk factors for the development of hypertension in patients treated with inhibitors of the VEGF signaling pathway. Hamnvik, O.P.,Cancer 121 (2), 311-319 (Jan 15, PubMed PMID: 25236375. Pubmed Central PMCID: 4293233).
Renal cell carcinoma: ESMO Clinical Practice Guidelines for diagnosis, treatment and follow-up; Escudier; Oxford JournalsMedicine & HealthAnnals of Oncology Volume 23 Issue suppl 7Pp. vii65-vii71
Differentiation of oncocytoma from chromophobe renal cell carcinoma (RCC): can novel molecular biomarkers help solve an old problem? Rajandram; J Clin Pathol 2014;67:97-104 doi:10.1136/jclinpath-2013-201895
Inter and Intratumour Heterogeneity: A Barrier to Individualized Medical Therapy in Renal Cell Carcinoma? Fischer K Opinion Article published: 18 May 2012 doi: 10.3389/fonc.2012.00049
Evaluation of Urine Aquaporin-1 and Perilipin-2 Concentrations as Biomarkers to Screen for Renal Cell Carcinoma A Prospective Cohort Study; Morrissey JJ; JAMA Oncol. 2015;1(2):204-212. doi:10.1001/jamaoncol.2015.0213
Urinary concentrations of aquaporin-1 and perilipin-2 in patients with renal cell carcinoma correlate with tumor size and stage but not grade. Morrissey JJ; Urology. 2014; 83(1):256.e9-256.e14.
Identification of a tumor-initiating stem cell population in human renal carcinomas. Bussolati,B.,Bruno,S.,Grange,C.,Ferrando,U.,andCamussi,G.(2008). FASEBJ. 22, 3696-3705.doi:10.1096/fj .08-102590
Identification of human brain tumour initiating cells; Singh; dNature 432, 396-401 (18 November 2004) | doi:10.1038/nature03128; Received 7 September 2004
Stem cell-like glioma cells promote tumor angiogenesis through vascular endothelial growth factor. Cancer Res. 2006 Aug 15;66(16):7843-8.

## Claims

1. A method for diagnosing renal cell carcinoma (RCC) in a subject comprising the steps of: i) measuring the expression level of soluble SEMA7A in a fluid sample; ii) comparing the expression measured at step i) with its predetermined reference value, and iii) concluding that the subject suffers from RCC when the expression level of SEMA7A is higher than its predetermined reference value or concluding that the subject does not suffer from RCC when the expression level of SEMA7A is lower than its predetermined reference value.

2. The method according to claim 1, wherein, the subject suffers or is susceptible of suffering from early stage of RCC.

3. An inhibitor of SEMA7A for use in the treatment of RCC in a subject in need thereof.

4. An inhibitor of SEMA7A for use according to claim 3 comprising: i) determining whether a subject suffers from early stages of RCC according to claim1 and ii) administrating to said subject a therapeutically amount of said inhibitor of SEMA7A when the expression level of SEMA7A is higher than its predetermined reference value.

5. A method of predicting the risk of having a relapse in a subject after a partial or total nephrectomy comprising the steps of: i) measuring the expression level of soluble SEMA7A in a fluid sample, ii) comparing the expression measured at step i) with its predetermined reference value, and iii) concluding that the subject has a risk of relapse when the expression level of SEMA7A is higher than its predetermined reference value or concluding that the subject has not a risk of relapse when the expression level of SEMA7A is lower than its predetermined reference value.

6. Use of a kit in the methods according to claims 1 to 2 and claim 5, wherein said kit comprises means for measuring the expression level of soluble SEMA7A in a fluid sample.

## Patentansprüche

1. Verfahren zum Diagnostizieren von Nierenzellkarzinom (RCC) in einer Versuchsperson, umfassend die Schritte des: i) Messens des Exprimierungsniveaus von löslichem SEMA7A in einer Fluidprobe; ii) Vergleichens der bei Schritt i) gemessenen Exprimierung mit ihrem vorher festgelegten Referenzwert und iii) Schlussfolgerns, dass die Versuchsperson an RCC leidet, wenn das Exprimierungsniveau von SEMA7A höher ist als sein vorher festgelegter Referenzwert, oder schlussfolgern, dass die Versuchsperson nicht an RCC leidet, wenn das Exprimierungsniveau von SEMA7A niedriger ist als sein vorher festgelegter Referenzwert.

2. Verfahren nach Anspruch 1, wobei die Versuchsperson an RCC im Frühstadium leidet oder dafür anfällig ist.

3. Inhibitor von SEMA7A zur Verwendung bei der Behandlung von RCC in einer Versuchsperson mit Bedarf daran.

4. Inhibitor von SEMA7A zur Verwendung nach Anspruch 3, umfassend: i) Bestimmen nach Anspruch 1, ob eine Versuchsperson an RCC im Frühstadium leidet und ii) Verabreichen einer therapeutischen Menge des Inhibitors von SEMA7A an die Versuchsperson, wenn das Exprimierungsniveau von SEMA7A höher ist als sein vorher festgelegter Referenzwert.

5. Verfahren zum Vorhersagen des Risikos bei einer Versuchsperson, nach einer teilweisen oder vollständigen Nephrektomie einen Rückfall aufzuweisen, umfassend die Schritte des: i) Messens des Exprimierungsniveaus von löslichem SEMA7A in einer Fluidprobe, ii) Vergleichens der bei Schritt i) gemessenen Exprimierung mit ihrem vorher festgelegten Referenzwert und iii) Schlussfolgerns, dass die Versuchsperson ein Risiko eines Rückfalls aufweist, wenn das Exprimierungsniveau von SEMA7A höher ist als sein vorher festgelegter Referenzwert, oder schlussfolgern, dass die Versuchsperson kein Risiko eines Rückfalls aufweist, wenn das Exprimierungsniveau von SEMA7A niedriger ist als der vorher festgelegte Referenzwert.

6. Verwendung eines Kits in den Verfahren nach Ansprüchen 1 bis 2 und Anspruch 5, wobei das Kit Mittel zum Messen des Exprimierungsniveaus von löslichem SEMA7A in einer Fluidprobe umfasst.

## Revendications

1. Procédé pour diagnostiquer un carcinome à cellules rénales (CCR) chez un sujet comprenant les étapes de : i) mesure du taux d'expression de SEMA7A soluble dans un échantillon liquide; ii) comparaison de l'expression mesurée à l'étape i) à sa valeur de référence prédéterminée, et iii) conclusion que le sujet souffre de CCR lorsque le taux d'expression de SEMA7A est supérieur à sa valeur de référence prédéterminée ou conclusion que le sujet ne souffre pas de CCR lorsque le taux d'expression de SEMA7A est inférieur à sa valeur de référence prédéterminée.

2. Procédé selon la revendication 1, dans lequel le sujet souffre ou est susceptible de souffrir d'un stade précoce de CCR.

3. Inhibiteur de SEMA7A à utiliser dans le traitement du CCR chez un patient dont l'état le nécessite.

4. Inhibiteur de SEMA7A à utiliser selon la revendication 3, comprenant : i) la détermination du fait qu'un sujet souffre ou non de stades précoces de CCR selon la revendication 1 et ii) l'administration audit sujet d'une quantité thérapeutique dudit inhibiteur de SEMA7A lorsque le taux d'expression de SEMA7A est supérieur à sa valeur de référence prédéterminée.

5. Procédé de prédiction du risque d'avoir une rechute chez un sujet après une néphrectomie partielle ou totale comprenant les étapes de : i) mesure du taux d'expression de SEMA7A soluble dans un échantillon liquide, ii) comparaison du taux d'expression mesuré à l'étape i) à sa valeur de référence prédéterminée, et iii) conclusion que le sujet présente un risque de rechute lorsque le taux d'expression de SEMA7A est supérieur à sa valeur de référence prédéterminée ou conclusion que le sujet ne présente aucun risque de rechute lorsque le taux d'expression de SEMA7A est inférieur à sa valeur de référence prédéterminée.

6. Utilisation d'un kit dans les procédés selon les revendications 1 à 2 et la revendication 5, dans laquelle ledit kit comprend un moyen pour mesurer le taux d'expression de SEMA7A soluble dans un échantillon liquide.
